# FASCICULE DE BREVET EUROPEEN

(11) **EP 1 935 488 B1**
(45) Date de publication et mention de la délivrance du brevet: **06.04.2016**
(21) Numéro de dépôt: 07291388.2
(22) Date de dépôt: 22.11.2007
(51) Int. Cl.: B01J 23/54, B01J 23/76, B01J 35/00, B01J 37/00, B01J 37/02, B01J 23/58, B01J 23/63, B01J 23/83

(54) **PROCÉDÉ DE PRÉPARATION D'UN CATALYSEUR PRÉPARÉ PAR IMPRÉGNATION D'UNE SOLUTION AQUEUSE CONTENANT DES PARTICULES SOUS FORME OXYDE, HYDROXYDE OU OXY(HYDROXY)DE D'UN CATION EN INTERACTION AVEC UNE ESPÈCE MOLÉCULAIRE D'UN MÉTAL DU GROUPE VIII ET PROCÉDÉ D'HYDROGÉNATION SELECTIVE AVEC CE CATALYSEUR**
VERFAHREN ZUR HERSTELLUNG EINES KATALYSATORS DURCH IMPRÄGNIERUNG MIT EINER WASSERLÖSUNG, DIE OXID-, HYDROXID- ODER OXY(HYDROXI)DTEILCHEN EINES KATIONS IN WECHSELWIRKUNG MIT EINER MOLEKULAREN SPEZIES EINES METALLS DER GRUPPE VIII UMFASST, SOWIE DESSEN VERWENDUNG ZUR SELEKTIVEN HYDROGENIERUNG
PROCESS FOR PREPARING A CATALYST BY IMPREGNATION WITH AN AQUEOUS SOLUTION CONTAINING OXIDE, HYDROXIDE OR OXY(HYDROXI)DE PARTICLES OF A CATION IN INTERACTION WITH A MOLECULAR SPECIES OF A GROUP VIII METAL AND USE THEREOF IN A SELECTIVE HYDROGENATION

(30) Priorité: 11.12.2006 FR 0610793
(43) Date de publication de la demande: 25.06.2008
(73) Titulaire: IFP Énergies nouvelles, 92852 Rueil-Malmaison Cedex (FR)
(72) Inventeur: Thomazeau, Cécile, 69530 Brignais (FR); Uzio, Denis, 69220 Belleville (FR); Verdon, Catherine, 69100 Villeurbanne (FR)

(56) Documents cités:
- EP-A- 0 979 673
- US-A- 4 590 177
- US-A- 4 764 499
- US-A1- 2001 036 902

## Description

Les procédés de conversion des hydrocarbures tels que le vaporeformage ou le craquage catalytique sont opérés à haute température et produisent une grande variété de molécules insaturées telles que l'éthylène, le propylène, les butènes linéaires, l'isobutène et les pentènes. En parallèle sont également formés des composés polyinsaturés contenant le même nombre d'atomes de carbone : acétylène, propadiène et méthylacétylène (ou propyne), 1-2 et 1-3 butadiène, vinylacétylène et éthylacétylène, et enfin d'autres composés polyinsaturés dont le point d'ébullition correspond à la fraction essence C5+. Tous ces composés polyinsaturés doivent être éliminés pour permettre l'utilisation de ces différentes coupes dans les procédés de pétrochimie tels que les unités de polymérisation.

Ainsi, par exemple, la coupe C2 de vapocraquage peut avoir la composition volumique moyenne suivante : 1,2% poids d'acétylène, 83,5% poids d'éthylène et 15,3% poids d'éthane.

Pour la coupe C3, on trouve le même type de répartition avec une prédominance du propylène (90% poids) et des teneurs en propadiène et méthylacétylène de l'ordre de 3 à 8% poids. Les spécifications concernant les concentrations de ces composés polyinsaturés pour les unités de pétrochimie et de polymérisation sont très basses : 20-30 ppm poids de MAPD (MéthylAcétylène et PropaDiène) pour le propylène qualité chimique et moins de 10 ppm poids voire jusqu'à 1 ppm poids pour la qualité « polymérisation ».

Une coupe C4 de vapocraquage aura par exemple une composition molaire moyenne suivante : 1 % de butane, 46,5% de butène, 51% de butadiène, 1,3% de VinylAcetylène (VAC) et 0,2% de butyne. La encore les spécifications sont sévères : teneur en dioléfines strictement inférieure à 10 ppm poids pour une coupe C4 qui sera utilisée en pétrochimie ou polymérisation.

Une coupe C5 de vapocraquage a par exemple une composition moyenne en masse suivante : 21% de pentanes, 45% de pentènes, 34% de pentadiènes.

Le procédé d'hydrogénation sélective s'est progressivement imposé pour éliminer les composés polyinsaturés des coupes pétrolières C2 à C5 citées car ce procédé permet la conversion des composés les plus insaturés vers les alcènes correspondants en évitant la saturation totale et donc la formation des alcanes correspondant, ceci pour les coupes C2 à C5 précitées.

L'hydrogénation sélective peut être réalisée en phase gaz ou liquide avec une préférence pour la phase liquide car cela permet d'abaisser le coût énergétique et d'augmenter la durée de cycle des catalyseurs. Les conditions opératoires pouvant être appliquées pour une réaction d'hydrogénation en phase liquide sont une pression comprise entre 1 et 3 MPa, de préférence une pression de 2 MPa, une température comprise entre 10 et 50°C et un ratio molaire hydrogène/(hydrocarbure à hydrogéner) compris entre 0,1 et 4, de préférence entre 1 et 2. La réaction d'hydrogénation sélective peut également être réalisée en phase gazeuse: dans ce cas, la pression peut être comprise entre 1 et 3 MPa, de préférence une pression de 2 MPa, la température peut être comprise entre 40°C et 120°C et le ratio molaire hydrogène/(hydrocarbure à hydrogéner) peut être compris entre 0,1 et 4, de préférence entre 1 et 2.

Des associations performantes de différents métaux ont été proposées pour améliorer les performances des catalyseurs d'hydrogénations sélectives.

Par exemple, le brevet US5356851 nous enseigne qu'il est avantageux d'associer un métal du groupe VIII (le groupe VIII selon la classification CAS correspond aux métaux des colonnes 8 à 10 selon la nouvelle classification IUPAC dans CRC Handbook of Chemistry and Physics, éditeur CRC press, rédacteur en chef D.R. Lide , 81^{ème} édition, 2000-2001), préférentiellement le Pd, à un élément tel que l'indium ou le gallium pour des applications en hydrogénation sélective de composés polyinsaturés De même des associations de plusieurs métaux permettent d'améliorer les performances des catalyseurs d'hydrogénation sélective. Nous pouvons citer les associations suivantes : Pd-Cu (US5464802), Pd-Ag (US4547600), Pd-Sn et Pd-Pb (JP59227829), Pd-Sn (EP0979673) ou encore une combinaison de Pd et d'un métal alcalin (EP0722776).

Ces effets bimétalliques sont en général liés à l'interaction créée entre les deux éléments métalliques en association. Il apparaît ainsi que l'identification d'un système catalytique multimétallique est conditionnée par l'établissement de cette interaction, donc par le contrôle de la composition de la particule. Ainsi, parmi les méthodes de synthèse permettant de contrôler les caractéristiques des particules bimétalliques en terme de composition, nous pouvons citer les méthodes de réactions contrôlées de surface (US20020045544, J Barbier, J.M. Dumas, C. Geron, H Hadrane Appl. Catal 179, 1994, 116 (1-2), S. Szabo, I. Bakos, F. Nagy, T. Mallat, J. Electroanal. Chem.1989, 263, 137) mettant notamment en jeu des phénomènes d'oxydo-réduction de surface.

### Résumé de l'invention

La présente invention décrit un procédé de préparation de ces précurseurs et de ces catalyseurs. Ces catalyseurs sont généralement composés de nanoparticules d'un métal du groupe VIII (le groupe VIII selon la classification CAS correspond aux métaux des colonnes 8 à 10 selon la nouvelle classification IUPAC dans CRC Handbook of Chemistry and Physics, éditeur CRC press, rédacteur en chef D.R. Lide , 81ème édition, 2000-2001), éventuellement associé à un deuxième métal. Ces nanoparticules métalliques sont associées à un oxy(hydroxy)de d'un cation choisi dans les colonnes IIA, IIIA, IIIB, IVB et IVA du tableau périodique (ces colonnes selon la classification CAS correspondent respectivement aux métaux de la colonne 2 pour la colonne IIA, aux métaux de la colonne 13 pour la colonne IIIA, aux métaux de la colonne 3 pour la colonne IIIB, aux métaux de la colonne 4 pour la colonne IVB et aux métaux de la colonne 14 pour la colonne IVA, selon la nouvelle classification IUPAC dans CRC Handbook of Chemistry and Physics, éditeur CRC press, rédacteur en chef D.R. Lide , 81ème édition, 2000-2001). Ces nanoparticules métalliques en association avec un oxy(hydroxyde) sont éventuellement supportées.

L'appellation oxy(hydroxyde) d'un cation désigne un cation des colonnes IIA, IIIA, IIIB, IVB et IVA qui peut être sous forme oxyde, sous forme hydroxyde ou sous une forme intermédiaire oxy(hydroxy)de. Par exemple, le cation Al³⁺ peut se trouver sous la forme d'un oxyde Al₂O₃, sous la forme d'un hydroxyde Al(OH)₃ ou sous une forme oxy(hydroxyde) AIO(OH).

Ces particules métalliques supportées peuvent posséder une taille moyenne comprise entre 1 et 5 nm. Cette taille est adaptée aux exigences des réactions d'hydrogénation sélective. En effet, la vitesse de réaction d'hydrogénation de molécules polyinsaturées telles que les dioléfines ou les acétyléniques dépend de la taille des particules métalliques. Ce résultat est généralement décrit sous le terme « sensibilité à la structure ». Un optimum est généralement observé pour une taille de l'ordre de 3 à 4 nm, cette valeur pouvant varier en fonction notamment de la masse moléculaire des réactifs (M. Boudart, W.C. Cheng, J. Catal. 106, 1987, 134, S. Hub, L. Hilaire, R. Touroude, Appl. Catal. 36 1992, 307). Il est ainsi généralement primordial d'obtenir une répartition en taille des particules centrée sur la valeur optimale ainsi qu'une dispersion minimale autour de cette valeur.

Par ailleurs, la répartition macroscopique des éléments (donc le métal du groupe VIII qui forme la nanoparticule métallique et le cation des colonnes IIA, IIIA, IIIB, IVB et IVA du tableau périodique dans le cas de la présente invention) dans le support constitue également un critère important, principalement dans le cadre de réactions rapides et consécutives telles que les hydrogénations sélectives. Il faut généralement que ces éléments se situent dans une croûte à la périphérie du support afin d'éviter les problèmes de transfert de matière intragranulaire pouvant conduire à des défauts d'activité et une perte de sélectivité. Ces particules formées d'un métal du groupe VIII, éventuellement associé à un deuxième métal, en association à un oxy(hydroxy)de d'un cation choisi dans les colonnes IIA, IIIA, IIIB, IVB et IVA du tableau périodique se situent donc généralement dans une croûte à la périphérie du support.

Dans la présente invention, de manière préférée, au moins 50% du métal, de façon préférée 75% du métal, de façon encore plus préférée 95% du métal, pourra être associé à l'oxy(hydroxy)de du cation des colonnes IIA, IIIA, IIIB, IVB et IVA du tableau périodique. Ces deux éléments peuvent former une croûte à la périphérie du support. L'épaisseur de cette croûte contenant à la fois le métal du groupe VIII et l'oxy(hydroxyde) du cation des colonnes IIA, IIIA, IIIB, IVB et IVA du tableau périodique peut être caractérisée par microsonde de Castaing. L'analyse par microsonde de Castaing permet généralement de déterminer le % poids des éléments présents dans une couronne à la périphérie du support comprise entre 0,1 R et R, de manière préférée comprise entre 0,5 R et R, de manière encore plus préférée entre 0,8 R et R, de manière très préférée entre 0,95 R et R. R est le rayon de la bille, de chaque cylindres du trilobe ou la moitié de la plus petite dimension de l'extrudé de support utilisé. Dans le cas de support mis en oeuvre sous forme de monolithe, R représente la demi épaisseur de la paroi.

Il est donc décrit ici une nouvelle voie de préparation de catalyseurs avec contrôle de la taille des particules métalliques et contrôle de la répartition du métal du groupe VIII et du cation des colonnes IIA, IIIA, IIIB, IVB et IVA du tableau périodique auquel il est associé, au sein de la bille de support.

L'association de ces deux éléments : métal du groupe VIII et cation des colonnes IIA, IIIA, IIIB, IVB et IVA du tableau périodique sous forme oxy(hydroxyde) dans une croûte à la périphérie des billes de support conduit à des catalyseurs dont les propriétés en hydrogénation sélective sont améliorées par rapports aux catalyseurs contenant des nanoparticules métalliques de un ou plusieurs métaux.

### Préparation des précurseurs

La méthode de préparation des précurseurs comporte les deux étapes suivantes (Étape 1 et Étape 2):

### Étape 1

Cette étape correspond, selon un premier mode de réalisation (étape 1a)), à la préparation d'une solution colloïdale A de pH défini contenant des particules oxy(hydroxy)de d'un cation M^{z+} sélectionné dans le groupe constitué par les cations des colonnes IIA, IIIA, IIIB, IVB et IVA de la classification périodique. Cette solution A est obtenue par dissolution, à une température comprise entre 5 et 100°C, du sel d'un précurseur du cation M^{z+} soluble en solution aqueuse, dans un volume V d'une solution de soude ayant une concentration de 0,01 à 1 moles/litres ou dans un volume V d'eau ou dans un volume V d'eau auquel est ajouté un volume V' d'une solution de soude ayant une concentration de 0,01 à 1 moles/litres .

Dans ce cas, les particules oxy(hydroxy)des du cation M^{z+} sont formées in-situ dans la solution par hydroxylation d'un précurseur cationique M(H₂O)ₙ^{z+} par une solution basique comme une solution d'hydroxyde de sodium (NaOH) ou d'hydroxyde de potassium (KOH). La réaction d'hydroxylation est la suivante :

M(H₂O)ₙ^{z+} + OH⁻ => M(OH)(H₂O)ₙ₋₁^{(z-1)+} + H₂O

z étant la charge formelle du cation comprise (bornes incluses) entre 1 et 4,
n étant la coordinence du cation comprise (bornes incluses) entre 1 et 6,
jusqu'à former le précurseur de charge nulle [M(OH)_{z}(H₂O)_{n-z}]⁰ qui est capable de se condenser par olation ou oxolation pour former des particules oxyde, hydroxyde ou oxyhydroxyde stables en solution dans des conditions de pH et de forces ioniques définies. Les particules seront généralement stable au moins jusqu'à l'étape 4 de séchage non incluse.

Cette étape correspond, selon un second mode de réalisation (étape 1b)), à l'utilisation d'une solution aqueuse colloïdale commerciale (solution A) de pH défini contenant des particules oxy(hydroxy)de d'un cation M^{z+} sélectionné dans le groupe constitué par les cations des colonnes IIA, IIIA, IIIB, IVB et IVA de la classification périodique. Dans ce cas, les particules oxy(hydroxy)des du cation M^{z+} sont formées ex-situ puis redispersées dans une solution aqueuse dans des conditions de pH et de force ioniques définies dans les quelles les particules oxy(hydroxy)des du cation M^{z+} sont stables. Les particules seront généralement stables au moins jusqu'à l'étape 4 de séchage non incluse

Le cation M^{z+} est choisi de façon préférée dans le groupe constitué par le magnésium pour la colonne IIA, le cérium pour la colonne IIIB, le titane ou le zirconium pour la colonne IVB, l'aluminium ou le galium pour la colonne IIIA et le silicium pour la colonne IVA.
Le cation M^{z+} est choisi de manière préférée dans le groupe constitué par les colonnes IIA et IIIB de la classification périodique. Le cation M^{z+} est choisi de manière très préférée dans le groupe constitué par le magnésium et le cérium. Le cation M^{z+} est de manière encore plus préférée le magnésium.

La source du cation M^{z+} peut être tout sel d'un précurseur du cation considéré soluble en solution aqueuse. Le sel du précurseur du cation M^{z+} peut être choisi dans le groupe constitué par un halogénure, un nitrate, un nitrite et un sulfate du cation. Un sel associant un halogénure, un nitrate, un nitrite ou un sulfate du cation avec un composé alcalin, avec un composé alcalino-terreux, avec un groupement amine ou avec un groupement ammoniac peut aussi être utilisé.

La concentration en cation M^{z+} dans la solution A peut être comprise entre 0,01 et 1 moles/litres, de façon préférée entre 0,05 et 0,5 moles/litres, de façon encore plus préférée entre 0,1 et 0,3 moles/litres.

Les particules oxy(hydroxy)de d'un cation M^{z+} peuvent avoir une taille comprise entre 1 nm et 1 µm, de préférence entre 10 et 100 nm.

Cette étape peut être effectuée à une température comprise entre 5 et 100°C, de façon préférée entre 20 et 80°C.

### Étape 2

Cette étape correspond au versement, de préférence en goutte à goutte, d'une solution aqueuse B contenant un sel précurseur d'un métal du groupe VIII, le sel précurseur du métal étant soluble dans les conditions de pH utilisées dans l'étape 1.

De façon préférée, le métal du groupe VIII est choisi dans le groupe constitué par le palladium, le platine, le cobalt et le nickel. De façon encore plus préférée, le métal engagé est choisi dans le groupe constitué par le platine et le palladium. De façon très préférée, le métal engagé est le palladium.

Le sel du précurseur du métal du groupe VIII peut être un sel d'un précurseur du métal considéré présentant un degré d'oxydation du métal supérieur à 0 et soluble en solution aqueuse. Le sel du précurseur du métal du groupe VIII peut être choisi dans le groupe constitué par un halogénure, un oxyde, un hydroxyde, un nitrate, un nitrite et un sulfate du métal, un sel associant un halogénure, un oxyde, un hydroxyde, un nitrate, un nitrite ou un sulfate du métal avec un composé alcalin, avec un alcalino-terreux, avec un groupement amine ou avec un groupement ammoniac.

Il peut être choisi de manière plus préférée dans le groupe constitué par le chlorure de palladium, le bromure de palladium, l'iodure de palladium, l'hexachloropalladate de potassium, l'hexachloropalladate d'ammonium, le tétrabromopalladate de potassium, le tétrachloropalladate de potassium, le tétrachloropalladate d'ammonium, l'hexachloropalladate de sodium, le tétrachloropalladate de sodium, le nitrate de palladium, le nitrite de palladium, le nitrite de diaminepalladium, le sulfate de palladium, le nitrate de tétraaminepalladium, le dichlorodiamine palladium et l'acétate de palladium.
La concentration du sel précurseur du métal du groupe VIII peut être comprise entre 0,001 et 1 mole/litre, de préférence entre 0,01 et 0,1 mole/litre, et de manière plus préférée entre 0,01 et 0,05 mole/litre.
L'étape 2 peut être éventuellement complétée par une étape de maturation de quelques minutes à plusieurs heures, de préférence de 10 minutes à 2 heures, de façon encore plus préférée de 30 minutes à 1 heure à une température comprise entre 5 et 100°C et de façon préférée entre 20 et 80°C.

### Préparation d'un catalyseur à partir du précurseur issu de l'étape 2

### Étape 3

C'est une étape d'imprégnation de la solution obtenue après l'étape 2 sur un support.

L'imprégnation avec le support peut être réalisée par imprégnation à sec ou en excès, en mode statique ou dynamique.

Le support peut comprendre au moins un oxyde réfractaire choisi dans le groupe constitué par les oxydes de magnésium, d'aluminium, de silicium, de zirconium, de thorium, ou de titane, pris seul ou en mélange entre eux ou avec d'autres oxydes de la classification périodique, tel que la silice-alumine. De préférence, le support est un oxyde d'aluminium (alumine) ou de silice. Le support peut également être un charbon, un silico-aluminate, une argile ou tout autre composé connu pour être utilisé comme support.

De préférence, le support présente une surface BET comprise entre 5 et 300 m²/g, de façon encore plus avantageuse entre 10 et 150m²/g.

Le support peut être mis en forme de billes, d'extrudés, de trilobe ou de monolithe

### Étape 4

Dans cette étape, le produit obtenu à l'étape 3 est séché, sous atmosphère inerte ou sous air, à une température inférieure ou égale à 200°C, de préférence inférieure ou égale à 120°C puis de calcination, sous atmosphère inerte ou sous air, à une température comprise entre 100 et 600°C de façon préférée entre 200 et 450°C.

Selon une variante, le procédé de préparation du catalyseur comprend en outre, après l'étape 4, un traitement d'activation du catalyseur sous atmosphère réductrice à une température comprise entre 100 et 600°C.

L'invention porte également sur les précurseurs préparés par l'enchaînement des étapes 1 et 2 suivit éventuellement d'une étape de maturation.

L'invention porte également sur les catalyseurs préparés selon l'enchaînement des étapes 1 à 4. Ces deux enchaînements peuvent être réalisés avec ou sans étape de maturation entre les étapes 2 et 3 et avec ou sans traitement d'activation à l'issue de l'étape 4.

A l'issue des étapes de préparation du catalyseur, (étape 3 et 4), la teneur en métal du groupe VIII est de préférence comprise entre 0,01 et 30 % en poids, de préférence entre 0,01 et 10 % en poids, de façon encore plus préférée entre 0,1 et 1% en poids. La teneur en cation des colonnes IIA, IIIA, IIIB, IVB et IVA est de préférence comprise entre 0,01 et 30 % en poids, de préférence entre 0,01 et 10 % en poids, de façon encore plus préférée entre 0,1 et 1% en poids.

Selon une variante de l'invention, la méthode de production d'un catalyseur à base de nanoparticules déposées sur un support peut comprendre, en outre, l'ajout d'au moins un élément choisi parmi:
- les métaux alcalins, de préférence le lithium, le sodium ou le potassium,
- les halogènes.
L'ajout éventuel d'au moins un métal alcalin peut être réalisé de manière à obtenir une teneur en métal alcalin dans le catalyseur comprise entre 0 et 20 % en poids, de préférence entre 0 et 10 % en poids, de manière plus préférée entre 0,01 et 5 % en poids.
L'ajout éventuel d'au moins un halogène peut être réalisé de manière à obtenir une teneur en halogènes dans le catalyseur comprise entre 0 et 0,2 % en poids.
Ces éléments sont ajoutés à l'issue de l'étape 2, à la solution contenant le métal du groupe VIII et l'oxy(hydroxy)de du cation des colonnes IIA, IIIA, IIIB, IVB et IVA, ou par imprégnation d'une solution contenant l'alcalin ou l'halogène, au catalyseur supporté à l'issue de l'étape 3 ou lors de l'étape 4 après l'étape de séchage et/ou après l'étape de calcination.

L'invention porte également sur un procédé d'hydrogénation sélective d'une coupe oléfinique mettant en oeuvre le catalyseur obtenu à l'issu de l'étape 4. De préférence, le catalyseur a subit un traitement d'activation avant son utilisation.

Le catalyseur obtenu selon la présente invention est utilisé pour l'hydrogénation sélective d'une coupe oléfinique. La coupe oléfinique peut être une coupe oléfinique légère contenant principalement des hydrocarbures à 3, 4 ou 5 atomes de carbones. A l'échelle industrielle, le catalyseur peut être, de préférence, mis en oeuvre en lit fixe à une température comprise entre 0°C et 200°C, de préférence entre 10°C et 200°C. Une pression suffisante est généralement requise pour maintenir au moins 80% poids de la coupe oléfinique en phase liquide à l'entrée du réacteur et une vitesse spatiale horaire (rapport entre le débit volumique de la charge oléfinique et le volume de catalyseur) comprise entre 1 et 50 h⁻¹. Cette pression est généralement comprise entre 0,3 et 6 MPa, de manière préférée entre 1 et 5 MPa, de façon encore plus préférée entre 1 et 3 MPa. L'hydrogénation sélective se fait, généralement, en présence d'une quantité d'hydrogène en faible excès par rapport à la valeur stoechiométrique requise pour l'hydrogénation des dioléfines et des acétyléniques. L'hydrogène et la charge oléfinique sont généralement introduits en courant ascendant ou descendant.

Selon une variante, le catalyseur obtenu selon la présente invention est utilisé pour l'hydrogénation sélective d'une coupe oléfinique, le catalyseur étant mis en oeuvre sur une coupe oléfinique en phase gaz, la pression étant comprise entre 1 et 3 MPa, la température étant comprise entre 40°C et 120°C et le ratio molaire hydrogène/(hydrocarbure à hydrogéner) étant compris entre 0,1 et 4. L'hydrogénation sélective se fait, généralement, en présence d'une quantité d'hydrogène en faible excès par rapport à la valeur stoechiométrique requise pour l'hydrogénation des dioléfines et des acétyléniques.

Pour des besoins d'évaluation à l'échelle du laboratoire ou à l'échelle pilote, on utilise généralement des réacteurs discontinus parfaitement agités de type Grignard. L'hydrogénation de la coupe oléfinique (par exemple, une coupe de 1,3-butadiène) peut être, de préférence, réalisée en phase liquide (par exemple, dans le n-heptane). La coupe oléfinique peut être une coupe oléfinique légère contenant principalement des hydrocarbures à 3, 4 ou 5 atomes de carbones. Le catalyseur peut être, de préférence, mis en oeuvre à une température comprise entre 0°C et 200°C, de préférence entre 10°C et 200°C. La pression est généralement comprise entre 0,3 et 6 MPa, de manière préférée entre 1 et 5 MPa, de façon encore plus préférée entre 1 et 3 MPa.

Les produits de la réaction peuvent être analysé par chromatographie en phase gazeuse. Les activités catalytiques sont exprimées en moles d'hydrogène (H₂) consommées par seconde et par atome de métal accessible aux réactifs. L'unité est le (moles H₂)/[s×(atome métal de surface)].
Les exemples qui suivent, non limitatifs, illustrent l'invention.

### Exemple 1 : Pd-Mg / Al₂O₃ (conforme à l'invention) - catalyseur A

Des particules d'hydroxyde de magnésium Mg(OH)₂ sont dans un premier temps formées dans une solution, nommée solution A. Pour cela, 3,6 g de nitrate de magnésium hexahydraté Mg(NO₃)₂, 6 H₂O sont dissous dans 50 ml d'une solution d'hydroxyde de sodium NaOH de concentration 0,5 N et de pH=11.

En parallèle une solution contenant le précurseur tetrahydroxopalladate Pd(OH)₄²⁻ est préparée. Elle est nommée solution B. Pour cela, 3,5 g d'une solution de nitrate de palladium Pd(NO₃)₂ contenant 8,5 % poids de palladium Pd sont prélevés et mis en solution dans 50 ml d'une solution d'hydroxyde de sodium NaOH de concentration 1 N et de pH=11.

La solution B est versée goutte à goutte dans la solution A. La solution obtenue possède un pH = 11 et est ensuite imprégnée sur une alumine du type δ - Al₂O₃ mise en forme sous forme de billes.

Le catalyseur est séché à 120°C puis est calciné à 200°C.

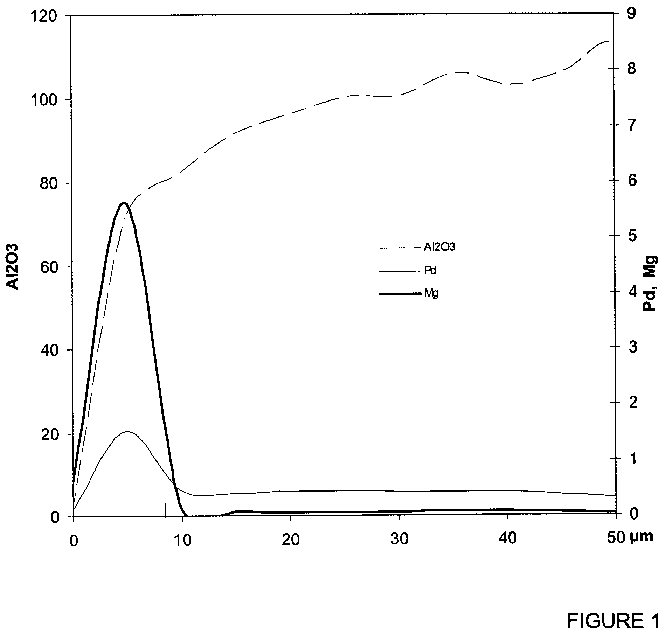

La caractérisation du catalyseur par microsonde de Castaing est donnée Figure 1. La figure 1 donne la répartition des éléments Pd et Mg dans la bille d'alumine pour le catalyseur A. Les abscisses sont données en micromètres (µm). Les ordonnées sont données en unité arbitraire.

Le catalyseur A obtenu contient 0,3% de magnésium Mg, 0,3% de palladium Pd et les éléments palladium et magnésium sont localisés sur une croûte d'une épaisseur égale à 15 µm.

### Exemple 2 : Catalyseur Pd-Mg / Al₂O₃ (non conforme à l'invention) - Catalyseur B.

5,3 g de nitrate de magnésium hexahydraté Mg(NO₃)₂, 6 H₂O sont dissous dans 50 ml d'eau (solution A). Le pH de la solution A est 6,5. Dans ces conditions, les particules de Mg(OH)₂ ne se forment pas. Ces particules ne sont pas stable dans ces conditions de pH.

En parallèle une solution contenant le précurseur nitrate de palladium Pd(NO₃)₂ est préparée. Pour cela, 3,5 g d'une solution de nitrate de palladium Pd(NO₃)₂ contenant 8,5 % poids de palladium Pd sont prélevés et mis en solution dans 50 ml d'une solution d'eau (solution B). Le pH de la solution B est 0,7.

La solution B est versée goutte à goutte dans la solution A. Le pH de la solution obtenue est 1.

La solution obtenue est ensuite imprégnée sur une alumine de type δ - Al₂O₃ mise en forme sous forme de billes.

Le catalyseur est séché à 120°C, puis est calciné à 200°C.

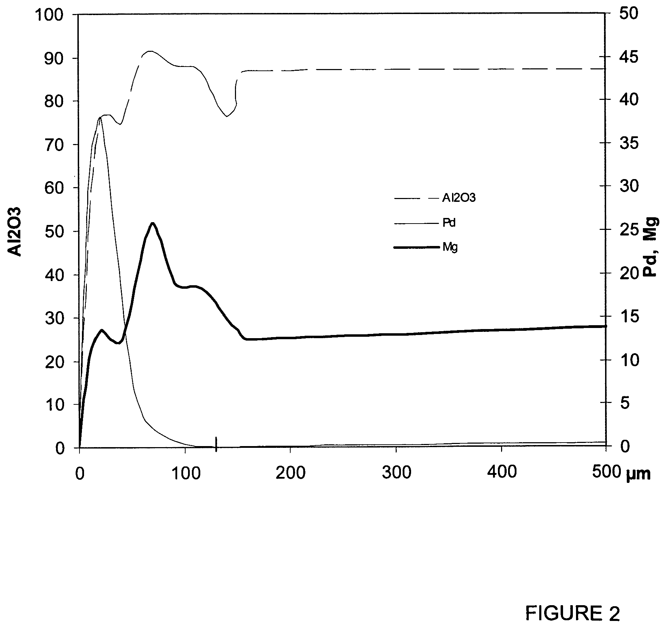

La caractérisation du catalyseur par microsonde de Castaing est donnée Figure 2. La figure 2 donne la répartition des éléments Pd et Mg dans la bille d'alumine pour le catalyseur B. Les abscisses sont données en micromètres (µm). Les ordonnées sont données en unité arbitraire.

Le catalyseur B obtenu contient 0,3% Mg, 0,3% Pd. L'élément Pd est localisé sur une croûte d'épaisseur 100 µm, l'élément Mg est présent sur toute la bille

### Exemple 3: Catalyseur Pd-Ce / Al₂O₃ (conforme à l'invention) - Catalyseur C.

2,5 g d'une solution aqueuse de pH=1,5 contenant des nanoparticules colloïdales de CeO₂ avec une concentration en CeO₂ de 20% poids sont mis en solution dans 50 ml d'eau (solution A). Le pH de la solution A est 3,4.

En parallèle une solution contenant le précurseur Pd(NO₃)₂ est préparée. Pour cela, 3,5 g d'une solution de Pd(NO₃)₂ contenant 8,5 % poids de Pd sont prélevés et mis en solution dans 50 ml d'eau (solution B). Le pH de la solution B est 0,7.

La solution B est versée goutte à goutte dans la solution A. La solution obtenue possède un pH de 1,3 et est ensuite imprégnée sur une alumine δ - Al₂O₃ mise en forme sous forme de billes.

Le catalyseur est séché à 120°C, calciné à 200°C.

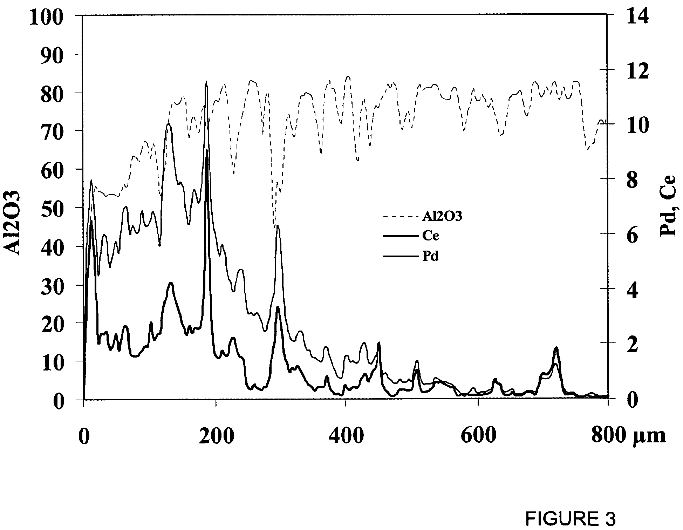

La caractérisation du catalyseur par microsonde de Castaing est donnée Figure 3. La figure 3 donne la répartition des éléments Pd et Ce dans la bille d'alumine pour le catalyseur C. Les abscisses sont données en micromètres (µm). Les ordonnées sont données en unité arbitraire.

Le catalyseur C obtenu contient 0,3% de cérium Ce et les éléments Pd et Ce localisés sur une croûte d'épaisseur 500 µm.

### Exemple 4 : Catalyseur Pd / Al₂O₃ (référence) - Catalyseur E

3,5 g d'une solution de Pd(NO₃)₂ contenant 8,5 % poids de Pd sont prélevés et mis en solution dans 100 ml d'eau.

Cette solution est imprégnée sur des billes d'alumine δ - Al₂O₃.

Le catalyseur est séché à 120°C, calciné à 200°C.

Le catalyseur D obtenu contient 0,3% Pd.

### Exemple 5 : Test catalytique en hydrogénation du 1,3-butadiène pour les catalyseurs à base de Pd

L'hydrogénation du 1,3-butadiène a été réalisée en phase liquide (n-heptane) dans un réacteur discontinu parfaitement agité de type « Grignard » sous une pression constante de 0,5 MPa d'hydrogène et une température de 5°C. Les produits de la réaction ont été analysés par chromatographie en phase gazeuse. Les activités catalytiques sont exprimées en moles de H₂ consommées par seconde et par atome de métal accessible aux réactifs et sont reportées dans le tableau 1. La sélectivités en 1-butène est donnée par les rapport des vitesses de la réaction d'hydrogénation du 1,3-butadiène (noté k_{(1,3-butadiène)}) et des vitesses de la réaction consécutive à l'hydrogénation du 1-butène (noté k_{(1-butène)}). Avant test, les catalyseurs ont préalablement été traités sous hydrogène à 150°C.

**Tableau 1: Activités et sélectivités mesurées en hydrogénation du 1,3-butadiène**

| | k_{(1,3-butadiène)} (moles H₂)/[s×(atome métal de surface)] | k_{(1,3-butadiène)}/k_{(1-butène)} (moles H₂)/[s×(atome métal de surface)] |
|---|---|---|
| Catalyseur A (selon invention) | 72 | 16 |
| Catalyseur B (non conforme) | 25 | 3 |
| Catalyseur C (selon invention) | 40 | 5 |
| Catalyseur D (selon référence) | 22 | 3 |

Le catalyseur B (non conforme à l'invention) possède une activité k_{(1,3-butadiène)} et une sélectivité en 1-butène (k_{(1,3-butadiène)}/k_{(1-butène)}) équivalentes à l'activité et la sélectivité du catalyseur D de référence.

Les catalyseurs A et C conformes à l'invention possèdent des activités et sélectivités améliorées par rapport à l'activité et la sélectivité du catalyseur D de référence.

## Revendications

1. Procédé de préparation d'un catalyseur comprenant les étapes suivantes:
1a) préparation d'une solution colloïdale A de pH défini contenant des particules sous forme oxyde, hydroxyde ou oxy(hydroxyde) d'un cation Mz+ sélectionné dans le groupe constitué par les cations des colonnes 2, 3, 4, 13 et 14 selon la classification périodique IUPAC dans CRC Handbook of Chemistry and Physics, 2000-2001, par dissolution à une température comprise entre 5 et 100°C du sel d'un précurseur du cation Mz+ soluble en solution aqueuse, la concentration en cation étant comprise entre 0,01 et 1 moles/litres dans un volume V d'une solution de soude ayant une concentration de 0,01 à 1 mole/litre ou dans un volume V d'eau ou dans un volume V d'eau auquel est ajouté un volume V' d'une solution de soude ayant une concentration de 0,01 à 1 moles/litres,
ou
1b) utilisation d'une solution aqueuse colloïdale commerciale (solution A) de pII défini contenant des particules sous forme oxyde, hydroxyde ou oxy(hydroxy)de d'un cation Mz+ sélectionné dans le groupe constitué par les cations des colonnes 2, 3, 4, 13 et 14 selon la classification périodique IUPAC dans CRC Handbook of Chemistry and Physics, 2000-2001
2) versement d'une solution aqueuse B contenant un sel précurseur d'un métal des colonnes 8 à 10 selon la classification périodique IUPAC dans CRC Handbook of Chemistry and Physics, 2000-2001, dont la concentration est de 0,001 à 1 moles/litres, le sel précurseur du métal étant soluble dans les conditions de pH utilisées dans l'étape 1.
3) imprégnation de la solution obtenue après l'étape 2 sur un support présentant une surface BET comprise entre 5 et 300 m²/g.
4) séchage à une température inférieure ou égale à 120°C puis de calcination à une température comprise entre 100 et 600°C.

2. Procédé selon la revendication 1 dans lequel le sel du précurseur du métal des colonnes 8 à 10 est un sel d'un précurseur du métal considéré présentant un degré d'oxydation du métal supérieur à 0 et soluble en solution aqueuse.

3. Procédé selon la revendication 2 dans lequel le sel du précurseur du métal des colonnes 8 à 10 est choisi dans le groupe constitué par un halogénure, un oxyde, un hydroxyde, un nitrate, un nitrite et un sulfate du métal, un sel associant un halogénure, un oxyde, un hydroxyde, un nitrate, un nitrite ou un sulfate du métal avec un composé alcalin, avec un alcalino-terreux, avec un groupement aminé ou avec un groupement ammoniac.

4. Procédé selon l'une des revendications précédentes dans lequel le sel du précurseur du métal des colonnes 8 à 10 est choisi dans le groupe constitué par le chlorure de palladium, le bromure de palladium, l'iodure de palladium, l'hexachloropalladate de potassium, l'hexachloropalladate d'ammonium, le tétrabromopalladate de potassium, le tétrachloropalladate de potassium, le tétrachloropalladate d'ammonium, l'hexachloropalladate de sodium, le tétrachloropalladate de sodium, le nitrate de palladium, le nitrite de palladium, le nitrite de diaminepalladium, le sulfate de palladium, le nitrate de tétraaminepalladium, le dichlorodiamine palladium et l'acétate de palladium.

5. Procédé selon l'une des revendications précédentes dans lequel la concentration du sel précurseur du métal des colonnes 8 à 10 est comprise entre 0,01 et 0,05 mole/litre.

6. Procédé selon l'une des revendications précédentes dans lequel le sel du précurseur du cation Mz+ est choisi dans le groupe constitué par un halogénure, un nitrate, un nitrite et un sulfate du cation, un sel associant un halogénure, un nitrate, un nitrite ou un sulfate du cation avec un composé alcalin, avec un composé alcalino-terreux, avec un groupement amine ou avec un groupement ammoniac.

7. Procédé selon l'une des revendications précédentes dans lequel la concentration en cation Mz+ dans la solution A est comprise entre 0,1 et 0,3 moles/litres.

8. Procédé selon l'une des revendications précédentes dans lequel le cation Mz+ est choisi dans le groupe constitué par les colonnes 2 et 3 de la classification périodique.

9. Procédé selon l'une des revendications précédentes dans lequel l'étape 2 est complétée par une maturation de quelques minutes à plusieurs heures à une température comprise entre 20 et 100°C.

10. Procédé selon l'une des revendications précédentes comprenant en outre après l'étape 4 un traitement d'activation du catalyseur sous atmosphère réductrice à une température comprise entre 100 et 600°C.

11. Procédé d'hydrogénation sélective d'une coupe oléfinique mettant en oeuvre le catalyseur obtenu par le procédé de préparation selon l'une des revendications précédentes.

12. Procédé d'hydrogénation sélective d'une coupe oléfinique selon la revendication 11 dans lequel le catalyseur est mis en oeuvre en lit. fixe à une température comprise entre 0°C et 200°C, la pression est suffisante pour maintenir au moins 80% poids de la coupe oléfinique à traiter en phase liquide à l'entrée du réacteur, la vitesse spatiale horaire est comprise entre 1 et 50 h-1, la pression est comprise entre 0,3 et 6 MPa.

13. Procédé d'hydrogénation sélective d'une coupe oléfinique selon la revendication 11 dans lequel le catalyseur est mis en oeuvre sur une coupe oléfinique en phase gaz, la pression étant comprise entre 1 et 3 MPa, la température étant comprise entre 40°C et 120°C et le ratio molaire hydrogène/(hydrocarbure à hydrogéner) étant compris entre 0,1 et 4.

## Patentansprüche

1. Verfahren zur Herstellung eines Katalysators, umfassend die folgenden Schritte:
1a) Herstellen einer kolloidalen Lösung A mit definiertem pH-Wert, enthaltend Oxid-, Hydroxid- oder Oxy(hydroxid)teilchen eines Kations Mz+, ausgewählt aus der Gruppe bestehend aus den Kationen der Spalten 2, 3, 4, 13 und 14 gemäß dem IUPAC-Periodensystem im CRC Handbook of Chemistry and Physics, 2000-2001, durch Auflösen bei einer Temperatur im Bereich zwischen 5 und 100 °C des Salzes eines Vorläufers des Kations Mz+, das in wässriger Lösung löslich ist, wobei die Kationenkonzentration im Bereich zwischen 0,01 und 1 Mol/Liter in einem Volumen V einer Natronlauge liegt, die eine Konzentration von 0,01 bis 1 Mol/Liter aufweist, oder in einem Volumen V von Wasser oder in einem Volumen V von Wasser, zu dem ein Volumen V' einer Natronlauge gegeben wurde, die eine Konzentration von 0,01 bis 1 Mol/Liter aufweist,
oder
1b) Verwenden einer kommerziellen, kolloidalen, wässrigen Lösung (Lösung A) mit definiertem pH-Wert, enthaltend Oxid-, Hydroxid- oder Oxy(hydroxid)teilchen eines Kations Mz+, ausgewählt aus der Gruppe bestehend aus den Kationen der Spalten 2, 3, 4, 13 und 14 gemäß dem IUPAC-Periodensystem im CRC Handbook of Chemistry and Physics, 2000-2001,
2) Zugießen einer wässrigen Lösung B, enthaltend ein Vorläufersalz eines Metalls der Spalten 8 bis 10 gemäß dem IUPAC-Periodensystem im CRC Handbook of Chemistry and Physics, 2000-2001, deren Konzentration 0,001 bis 1 Mol/Liter beträgt, wobei das Vorläufersalz des Metalls unter den pH-Bedingungen löslich ist, die in Schritt 1 verwendet werden.
3) Imprägnieren mit der in Schritt 2 erhaltenen Lösung eines Trägers, der eine BET-Oberfläche im Bereich zwischen 5 und 300 m²/g aufweist.
4) Trocknen bei einer Temperatur kleiner oder gleich 120°C, dann Kalzinieren bei einer Temperatur im Bereich zwischen 100 und 600 °C.

2. Verfahren nach Anspruch 1, wobei das Salz des Vorläufers des Metalls der Spalten 8 bis 10 ein Salz eines Vorläufers des betrachteten Metalls ist, das eine Oxidationszahl des Metalls größer als 0 aufweist und in wässriger Lösung löslich ist.

3. Verfahren nach Anspruch 2, wobei das Salz des Vorläufers des Metalls der Spalten 8 bis 10 ausgewählt ist aus der Gruppe bestehend aus einem Halogenid, einem Oxid, einem Hydroxid, einem Nitrat, einem Nitrit und einem Sulfat des Metalls, wobei ein Salz ein Halogenid, ein Oxid, ein Hydroxid, ein Nitrat, ein Nitrit oder ein Sulfat des Metalls mit einer alkalischen Verbindung mit einer erdalkalischen Verbindung, mit einer Amingruppe oder mit einer Ammoniakgruppe assoziiert.

4. Verfahren nach einem der vorhergehenden Ansprüche, wobei das Salz des Vorläufers des Metalls der Spalten 8 bis 10 ausgewählt ist aus der Gruppe bestehend aus Palladiumchlorid, Palladiumbromid, Palladiumiodid, Kaliumhexachloropalladat, Ammoniumhexachloropalladat, Kaliumtetrabromopalladat, Kaliumtetrachloropalladat, Ammoniumtetrachloropalladat, Natriumhexachloropalladat, Natriumtetrachloropalladat, Palladiumnitrat, Palladiumnitrit, Diaminpalladiumnitrit, Palladiumsulfat, Tetraaminpalladiumnitrat, Palladiumdichlorodiamin und Palladiumacetat.

5. Verfahren nach einem der vorhergehenden Ansprüche, wobei die Konzentration des Vorläufersalzes des Metalls der Spalten 8 bis 10 im Bereich zwischen 0,01 und 0,05 Mol/Liter liegt.

6. Verfahren nach einem der vorhergehenden Ansprüche, wobei das Salz des Vorläufers des Kations Mz+ ausgewählt ist aus der Gruppe bestehend aus einem Halogenid, einem Nitrat, einem Nitrit und einem Sulfat des Kations, wobei ein Salz ein Halogenid, ein Nitrat, ein Nitrit oder ein Sulfat des Kations mit einer alkalischen Verbindung, mit einer erdalkalischen Verbindung, mit einer Amingruppe oder mit einer Ammoniakgruppe assoziiert.

7. Verfahren nach einem der vorhergehenden Ansprüche, wobei die Konzentration an Kation Mz+ in der Lösung A im Bereich zwischen 0,1 und 0,3 Mol/Liter liegt.

8. Verfahren nach einem der vorhergehenden Ansprüche, wobei das Kation Mz+ ausgewählt ist aus der Gruppe bestehend aus den Spalten 2 und 3 des Periodensystems.

9. Verfahren nach einem der vorhergehenden Ansprüche, wobei Schritt 2 durch eine Reifung von einigen Minuten bis mehreren Stunden bei einer Temperatur im Bereich zwischen 20 und 100 °C abgeschlossen wird.

10. Verfahren nach einem der vorhergehenden Ansprüche, umfassend ferner nach Schritt 4 eine Behandlung zur Aktivierung des Katalysators unter reduktiver Atmosphäre bei einer Temperatur im Bereich zwischen 100 und 600 °C.

11. Verfahren zur selektiven Hydrogenierung eines Olefinschnitts, bei dem der Katalysator eingesetzt wird, der mit dem Herstellungsverfahren nach einem der vorhergehenden Ansprüche erhalten wurde.

12. Verfahren zur selektiven Hydrogenierung eines Olefinschnitts nach Anspruch 11, wobei der Katalysator im Festbett bei einer Temperatur im Bereich zwischen 0 °C und 200°C eingesetzt wird, der Druck ausreicht, um mindestens 80 Gew.-% des zu behandelnden Olefinschnitts am Eingang des Reaktors in flüssiger Phase zu halten, die Raumgeschwindigkeit pro Stunde im Bereich zwischen 1 und 50 h-1 liegt, der Druck im Bereich zwischen 0,3 und 6 MPa liegt.

13. Verfahren zur selektiven Hydrogenierung eines Olefinschnitts nach Anspruch 11, wobei der Katalysator an einem Olefinschnitt in Gasphase eingesetzt wird, wobei der Druck im Bereich zwischen 1 und 3 MPa liegt, wobei die Temperatur im Bereich zwischen 40 °C und 120 °C liegt und wobei das Molverhältnis zwischen Wasserstoff und zu hydrierendem Kohlenwasserstoff im Bereich zwischen 0,1 und 4 liegt.

## Claims

1. A process for preparing a catalyst comprising the following steps:
1)a) preparing a colloidal solution A with a defined pH containing particles in the form of an oxide, hydroxide or (hydroxide) of a cation M^{z+} selected from the group constituted by cations from columns 2, 3, 4, 13 and 14 according to the IUPAC periodic classification in the CRC Handbook of Chemistry and Physics, 2000-2001 by dissolving, at a temperature in the range 5°C to 100°C, a salt of a precursor of the cation M^{z+} which is soluble in aqueous solution, the concentration of the cation being in the range 0.01 to 1 mole/litre, in a volume V of a solution of sodium hydroxide with a concentration of 0.01 to 1 mole/litre or in a volume V of water or in a volume V of water to which a volume V' of a solution of sodium hydroxide with a concentration of 0.01 to 1 mole/litre has been added;
1)b) using a commercial aqueous colloidal solution (solution A) with a defined pH containing particles in the form of an oxide, hydroxide or (hydroxide) of a cation M^{z+} selected from the group constituted by cations from columns 2, 3, 4, 13 and 14 according to the IUPAC periodic classification in the CRC Handbook of Chemistry and Physics, 2000-2001;
2) adding in an aqueous solution B containing a precursor salt of a metal of columns 8 to 10 according to the IUPAC periodic classification in the CRC Handbook of Chemistry and Physics, 2000-2001 with a concentration of 0.001 to 1 mole/litre, the precursor salt of the metal being soluble under the pH conditions used in step 1,
3) impregnating the solution obtained after step 2 onto a support with a BET specific surface area in the range 5 to 300 m²/g;
4) drying at a temperature of 120°C or less then calcining at a temperature in the range 100°C to 600°C.

2. A process according to claim 1, in which the precursor salt of the metal of columns 8 to 10 is a salt of a precursor of the metal under consideration having a metal oxidation number of more than 0 and which is soluble in aqueous solution.

3. A process according to claim 2, in which the precursor salt of the metal of columns 8 to 10 is selected from the group constituted by a halide, an oxide, a hydroxide, a nitrate, a nitrite and a sulphate of the metal, a salt associating a halide, an oxide, a hydroxide, a nitrate, a nitrite or a sulphate of the metal with an alkali compound, with an alkaline-earth, with an amine group or with an ammonia group.

4. A process according to one of the preceding claims, in which the precursor salt of the metal of columns 8 to 10 is selected from the group constituted by palladium chloride, palladium bromide, palladium iodide, potassium hexachloropalladate, ammonium hexachloropalladate, potassium tetrabromopalladate, potassium tetrachloropalladate, ammonium tetrachloropalladate, sodium hexachloropalladate, sodium tetrachloropalladate, palladium nitrate, palladium nitrite, diamine palladium nitrite, palladium sulphate, tetramine palladium nitrate, palladium dichlorodiamine and palladium acetate.

5. A process according to one of the preceding claims, in which the concentration of the precursor salt of the metal of columns 8 to 10 is in the range 0.01 to 0.05 mole/litre.

6. A process according to one of the preceding claims, in which the precursor salt of the cation M^{z+} is selected from the group constituted by a halide, a nitrate, a nitrite and a sulphate of the cation, a salt associating a halide, a nitrate, a nitrite or a sulphate of the cation with an alkali compound, an alkaline-earth compound, with an amine group or with an ammonia group.

7. A process according to one of the preceding claims, in which the concentration of the M^{z+} cation in solution A is in the range 0.1 to 0.3 mole/litre.

8. A process according to one of the preceding claims, in which the cation M^{z+} is selected from the group constituted by columns 2 and 3 of the periodic table.

9. A process according to one of the preceding claims, in which step 2 is completed by maturation for a few minutes to several hours at a temperature in the range 20°C to 100°C.

10. A process according to one of the preceding claims, further comprising after step 4, a catalyst activation treatment in a reducing atmosphere at a temperature in the range 100°C to 600°C.

11. A process for selective hydrogenation of an olefinic cut using the catalyst obtained by a preparation process according to one of the preceding claims.

12. A process for selective hydrogenation of an olefinic cut according to claim 11, in which the catalyst is employed in a fixed bed at a temperature in the range 0°C to 200°C, the pressure is sufficient to maintain at least 80% by weight of the olefinic cut to be treated in the liquid phase at the reactor inlet, the hourly space velocity is in the range 1 to 50 h⁻¹, and the pressure is in the range 0.3 to 6 MPa.

13. A process for selective hydrogenation of an olefinic cut according to claim 11, in which the catalyst is employed on an olefinic cut in the gas phase, the pressure being in the range 1 to 3 MPa, the temperature being in the range 40°C to 120°C and the hydrogen/(hydrocarbon to be hydrogenated) molar ratio being in the range 0.1 to 4.
